# EUROPEAN PATENT SPECIFICATION

(11) **EP 3 905 908 B1**
(45) Date of publication and mention of the grant of the patent: **08.01.2025**
(21) Application number: 19933337.8
(22) Date of filing: 20.06.2019
(51) Int. Cl.: A24F 40/05, A24F 40/10, A24F 40/40, A24F 40/42, A24F 40/44, A24F 40/485, A24F 40/50, A24F 40/53, A24F 40/60, A24F 40/65, A61M 15/06, B05B 17/06, A61M 11/00, A61M 11/04, A61M 15/00

(54) **PERSONAL ULTRASONIC ATOMIZER DEVICE**
PERSÖNLICHE ULTRASCHALLZERSTÄUBERVORRICHTUNG
DISPOSITIF D'ATOMISATION À ULTRASONS PERSONNEL

(43) Date of publication of application: 10.11.2021
(73) Proprietor: Shaheen Innovations Holding Limited, Abu Dhabi (AE)
(72) Inventor: ALSHAIBA SALEH GHANNAM ALMAZROUEI, Mohammed, Abu Dhabi (AE); YAMAN, Mohamad, Abu Dhabi (AE)
(74) Representative: Forresters IP LLP
(86) International application number: PCT/IB2019/055192
(87) International publication number: WO 2020/254862

(56) References cited:
- EP-A1- 3 434 118
- WO-A1-2017/108268
- WO-A1-2020/225534
- US-A1- 2017 135 411

## Description

### FIELD OF THE INVENTION

The invention relates to an ultrasonic atomizer device for personal use, and more particularly to an atomizer device for atomizing a liquid using ultrasonic oscillation of piezoelectric materials.

### BACKGROUND OF THE INVENTION

The harmful effects of smoking and tobacco products have been known for many years, and while a prevalent cause of disease and even death, most tobacco products continue to be sold and used worldwide.

Deemed a 'safer' alternative to smoking and smoking products, electronic smoking devices have been developed. The electronic devices use heating coils to convert a nicotine-based liquid to a fine mist or vape. The vape is then inhaled by the user in a similar fashion to smoking a cigarette. These devices have however, failed to eliminate all harmful components from the aerosol. Therefore, the correct terminology for such devices is 'reduced' risk products.

Increasingly studies have shown that the harmful side-effects of smoking, including heart, lung and mouth cancer, can be related to the combustion of the tobacco products, such as dry tobacco or nicotine-based liquids, using heat and/or an open flame. During this process the chemical nature of the compounds are changed, and it is this change which has been closely associated with the carcinogenic effects of smoking. Further, burning nicotine basedliquids, liquids or E-liquids as they are known, produces formaldehyde and acrolein. These are compounds which are known to be toxin to the body.

To avoid combustion, and the chemical change which takes place as a result, devices utilizing liquid cavitation have replaced heating and/or combustion. Oscillation of piezoelectric materials is used to cavitate nicotine-based liquids placed in contact therewith. The cavitation has the same effect of generating a mist or vape, similar to that of the combustible method but without the harmful chemical change in the compounds.

While devices utilizing this cavitation method have begun entering the market place, such devices still suffer from problems, making their introduction to, and uptake by, users undesirable. The devices which are entering the market are prone to leaking, function only in a particular orientation and have little or no external control. Such as control through other electronic devices.

The lack of connectivity of the devices lacks the control needed to monitor nicotine consumption. Thus the devices cannot be used as medical smoke cessation devices.

WO 2017/108268A1 discloses an electrically operated aerosol-generating system.

Some examples of the present disclosure seek to address these problems, at least partly, and provide an electronic smoking device which does not use combustion or heat to generate a vape, and which is leak-proof, usable in any orientation, including leak-proof use while inverted, and which may be controllable, remotely, through connection with an electronic device. The controllable nature making the device a candidate for medical smoke cessation.

### SUMMARY OF THE INVENTION

The present invention provides a personal ultrasonic atomizer device, as claimed in claim 1.

### BRIEF DESCRIPTION OF THE DRAWINGS

These and other features of the invention will become more apparent by the following description of the embodiment, which is made by way of example, with reference to the accompanying drawings in which:
- Figure 1:: shows a personal ultrasonic atomizer device in perspective view, in accordance with the invention;
- Figure 2:: shows a view from a side and in cross-section of the cartridge in accordance with the invention;
- Figure 3:: shows end views of the cartridge of Figure 2;
- Figure 4:: shows an enlarged view, in cross-section, of a valve arrangement in accordance with the invention;
- Figure 5:: shows a view in perspective of a member in accordance with the invention;
- Figure 6:: shows a perspective view, in partial cross-section, of a sonication chamber in accordance with the invention;
- Figure 7:: shows a view from a side and in cross-section of the device of Figure 1;
- Figure 8:: shows the personal ultrasonic atomizer device in accordance with the invention in an exploded view; and
- Figure 9:: shows a flow diagram depicting the operation of the device in accordance with the invention.

### BRIEF DESCRIPTION OF THE PREFERRED EMBODIMENTS

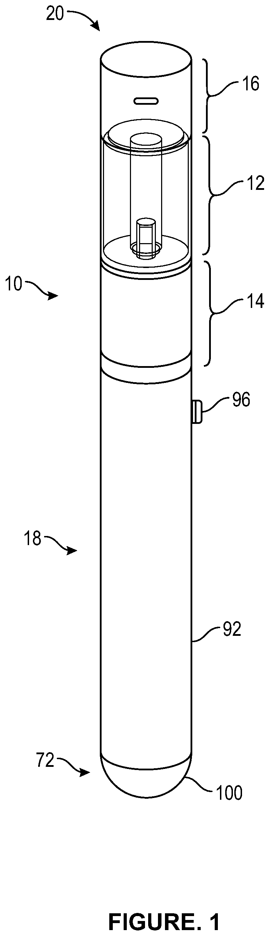

In accordance with the invention there is provided a personal ultrasonic atomizer device 10 as shown in Figure 1.

The device 10 includes a cartridge 12 for holding a liquid to be atomized, a sonication chamber 14 and a member 16 for controlling the amount of liquid flow to the chamber. The cartridge, chamber and member are interconnected in sequential fashion, with the cartridge flanked on either side by the chamber and the member.

The device 10 includes a casing 18 for housing components used to power and operate the device.

The device 10 is shown as an elongate tubular body which includes an end 20, an opposing end 22 and a sidewall 24 which partially extends between the first and second end.

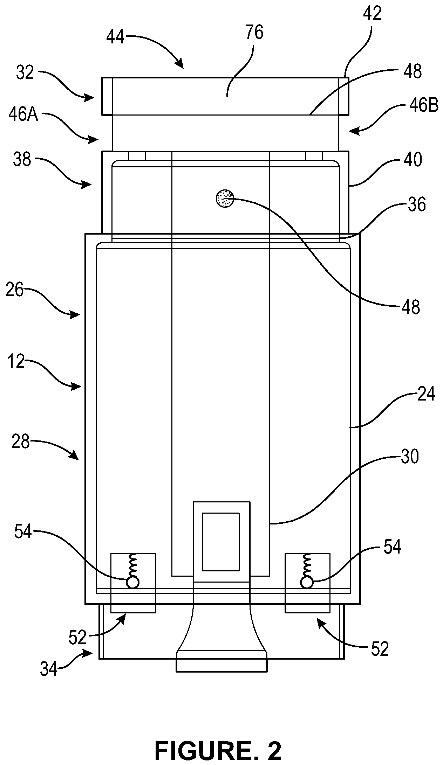

Figure 2 shows the cartridge 12 which includes a reservoir 26 for holding the liquid to be atomized. In the embodiment shown, the cartridge is an elongate circular shaped body 28 having an internal bore 30, which extends between a first end 32 and an opposed second end 34 of the body.

The body 28 includes a shoulder 36, at which the diameter of the body is reduced to form a portion 38. The portion includes a sidewall 40 which extends away from the first end 32 to terminate in a circular ridge 42. A recess 44 is formed in the space between the sidewall and the ridge.

The sidewall 40 includes a first duct 46A and a diametrically opposed second duct 46B, which both extend through the sidewall. Additionally, at least a first guide formation 48, extends laterally from the sidewall.

The portion 38 provides a mating arrangement for connecting the member 16.

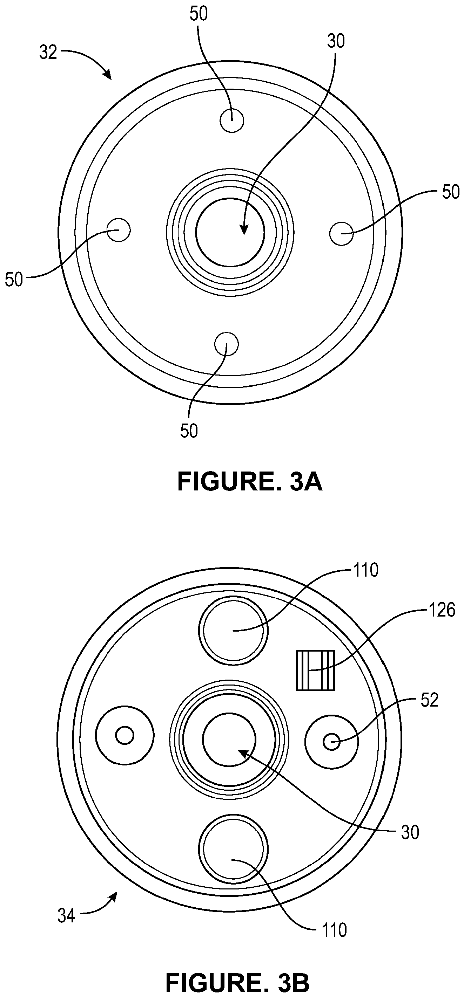

The cartridge 12 additionally includes at least a first opening 50, located along the first end 32 and at least a first aperture 52 located along the second end 34. This is more clearly shown in Figures 3A and 3B, respectively.

Situated along the second end 34 may be one or more metal plates 110 and a microchip 126. The metal plates form part of a complimentary arrangement for engaging the cartridge 12 with the sonication chamber 14 and will be dealt with in further detail below. Likewise, the microchip will also be discussed in greater detail below.

Opening 50 provides a passageway from the surroundings to the reservoir 26. In the current embodiment the cartridge 12 includes four such openings, all of which provide a passageway from the surroundings to the reservoir.

The at least first aperture 52, provides a flow path 54 from the reservoir 26 out of the cartridge 12. As is shown in Figure 3B. A second aperture is also present. The invention is not deemed to be limited as to the number of apertures in this respect.

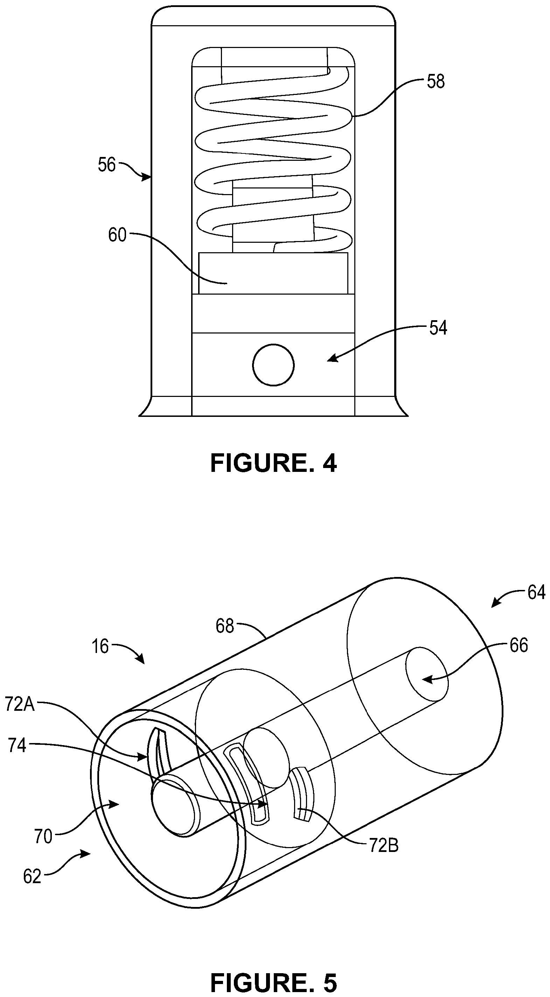

Apertures 52 each include a valve arrangement 56. An enlarged view of the valve arrangement is shown in Figure 4. The arrangement includes a biasing means 58, such as a mechanical spring, and a stopper 60 for sealing the aperture 52, when biased toward the sealing position. The flow path 54 will be restricted when the stopper is in the sealing position.

Turning to Figure 5 which shows the member 16. The member is formed as an elongate circular body and includes a leading end 62, a trailing end 64, an internal orifice 66, which extends between the leading end and trailing end, and an outerwall 68 which extends between the two ends.

Positioned toward the leading end 62 is an internal step 70, which step is compatible to receive portion 38 of the cartridge 12. The step additionally includes a pair of opposing slots 72A and 72B respectively, and at least one groove 74, for receiving the guide formation 48.

The member 16 is connected to the cartridge 12, by portion 38 being received by internal step 70. The cartridge 12 is inserted into the member until shoulder 36 abuts the leading end 62 of the member, and the guide formation 48 is received within the groove 74.

Once connected, the member 16 is movable relative to the cartridge 12. The movement may be in the form of a twist, as shown in the Figures, where the degree of movement will be limited, to an extent, by the movement of the guide formation 48 as it travels through groove 74.

Twisting the member 16 relative to the cartridge 12 results in slots 72A and 72B becoming aligned, or misaligned, with the ducts 46A and 46B, respectively. Alignment of the slots with the ducts will provide a passageway for air from the surroundings to the recess 44, and hence, to openings 50. This alignment relates to a flow position.

The degree of twisting movement of the member 16 relative to the cartridge 12, is limited to the degree of movement of the guide formation 48 as it travels through groove 74. This movement may also correlate to the alignment or misalignment of the slots 72 with the ducts 46. Mis-alignment relates to a sealing position.

The member 16, once connected to the cartridge 12, and twisted such that the slots 72A and 72B are misaligned with the ducts 46A and 46B, provides a substantially air-tight seal.

While the twisting of the member 16, relative to the cartridge 12, is described to align the slots 72 with the ducts 46, it is clear that any movement of the member in which the alignment takes place may be incorporated in terms of the current description. The invention is not deemed to be limited in this respect. Further the member 16 may be connected to the cartridge 12 in any fashion, wherein movement, relative to the cartridge, will result in the member aligning or misaligning the slots 72 with the ducts 46. The connection may include vertical, or lateral movement of the member.

Located within the recess 44 is a ring-shaped liquid retention means 76. The means, once inserted into the recess will cover the slots 72A and 72B of the member 16. The means will further cover the openings 50 of the cartridge 12. The means is substantially pervious to air but, is substantially impervious to a liquid.

When in use, the retention means 76 will provide a buffer between the surroundings and the openings 50. Liquid held in the reservoir 26 which may pass through the openings will be retained by the means, and hence is prevented from passing through the ducts 46, and/or, slots 72.

The retention means 76 provides an anti-leak guard, which limits movement of the liquid from the reservoir 26 and pass the openings. Placing the cartridge 12 and/or reservoir 26 in any orientation will have little or no effect on the anti-leak properties.

The retention means 76 provides for the device 10 to be used in any orientation, without the liquid being leaked out of the reservoir 26 and/or the device itself.

This is particularly useful where a user of the device may be horizontal (i.e. lying down). The device may be used in the horizontal position without any leakage of liquid.

The flow of air through the retention means 76 will not be restricted, to a degree. As such air from the surroundings may flow through the retention device, through the openings 50 and to reservoir 26. This will be the position when the member 16 is twisted to align the slots 72 with the ducts 46.

The flow of air will be limited, substantially, when the member 16 is twisted to misalign the slots 72 and ducts 46. Once misaligned the member and cartridge 12 will provide a substantial air-tight seal between them, thus limiting liquid and air-flow from the surroundings to the reservoir 26.

A vacuum within the reservoir 26 is formed when the cartridge 12 and the member 16 are connected to one another, and the member is set to the sealing position. The vacuum prevents the liquid held in the reservoir 26, from flowing through the apertures 50 (this is true even when the valve arrangement 56 is in a flow position).

To release the vacuum, air from the surroundings must be introduced to the reservoir 26. This is possible by moving the member 16 from the sealing potion to the flow position. Air may then flow through the slots 72 and the duct 46 through the openings 50 to the reservoir. By releasing the vacuum, liquid in the reservoir may flow out of the apertures 52.

The vacuum may be reinstated by moving the member 16 to the sealing position and restricting the air flow.

The rate at which liquid flows from the reservoir through the apertures may also be controlled, to a degree, by the amount of air being introduced. Controlling the air flow is possible by the limiting the degree to which the slots 72 and ducts 46 are aligned and/or misaligned, through the movement of the member 16 relative to the cartridge 12.

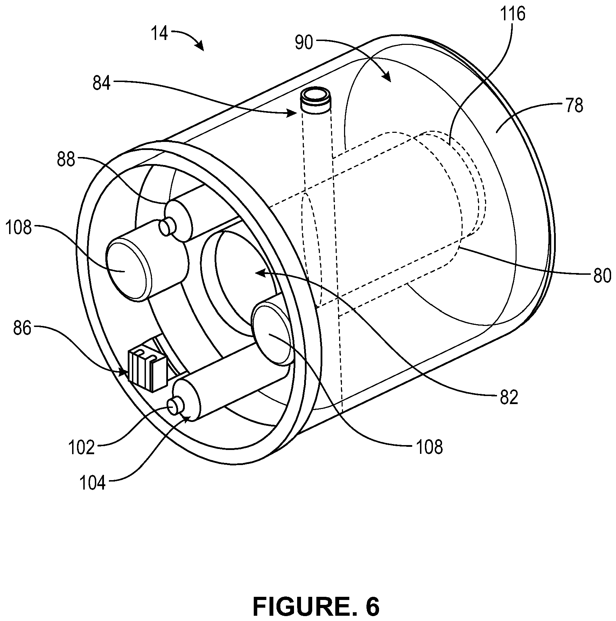

Turning to Figure 6, which shows the sonication chamber 14. The chamber includes the ultrasonic oscillation arrangement 78, provided for atomizing a liquid. The chamber further includes a wick 80, a portion of an inhalation channel 82, an air inlet port 84, a contact panel 86, a rigid connector 88 and an inner chamber 90.

Casing 18 houses a portion of the chamber 12 and includes an outer shell 92, conterminous with the outerwall 24 of the cartridge 12 and the sidewall 68 of the member 16.

The casing 18 houses additional components for powering and operating the device 10.

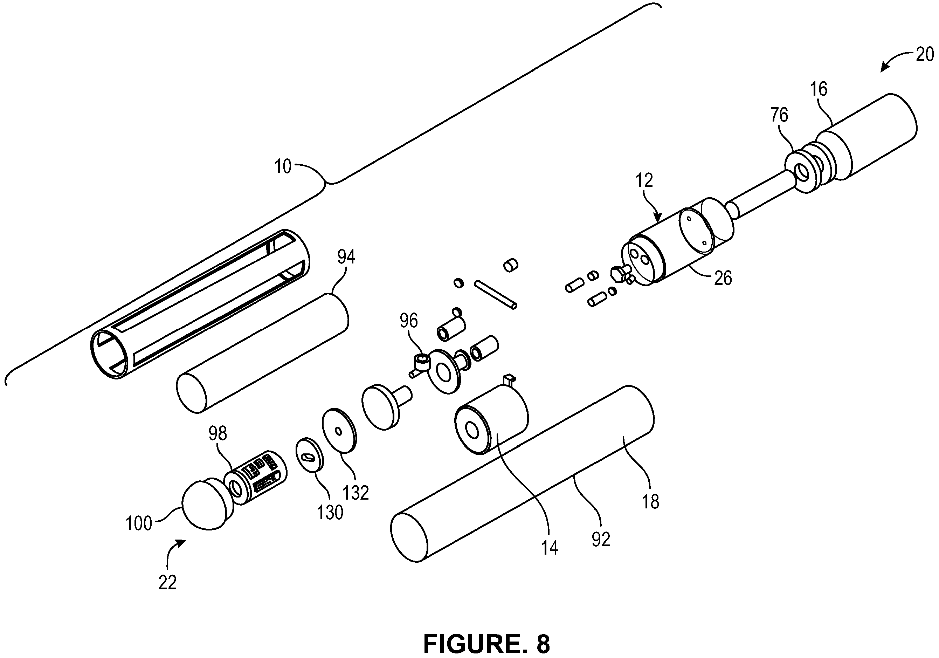

These components include an energy storage arrangement 94, activation switch 96, computer 98 and a visual indicator 100, such as a light emitting diode, for providing signals to a user. These are more clearly shown in the exploded view of the device 10 in Figure 8.

The chamber 14 includes two rigid connectors 88. Each of the connectors include a rigid piston 102 and a hole 104 and fulfil two roles in the invention. The first of these roles is to provide a liquid path 106 for a liquid from outside of the chamber, through the hole to an inner chamber 90, housed within the chamber. A liquid to be atomized is held in the inner chamber before atomization takes place. The inner chamber places the liquid in fluid communication with the wick 80.

The second role of the connectors is to move the stoppers 60 of the valve arrangements 56 from the sealing position, to an open position, to open the liquid path 106. This is achieved once the pistons 102 are brought into abutment with the valves, during engagement of the cartridge 12 and the chamber 14. Thus, liquid held in the reservoir 26 is permitted to flow over the pistons and through the holes 104, to inner chamber 90. Disengagement of the cartridge from the chamber provides the stoppers 60 of the valve arrangements to return, under bias, to the sealing position, effectively closing the liquid path 106 and sealing the reservoir.

The engageable nature of the cartridge 12 and the chamber 14, is made possible by a complimentary arrangement situated at an interface between the chamber and cartridge.

In the current embodiment, the chamber 14 includes a pair of magnets 108 spaced apart and aligned to attract a pair of metal plates 110 positioned along the cartridge 12. Bringing the magnets and plates into proximity allows the attractive force of the magnet to draw the plates into abutment, to form the engagement.

The complimentary arrangement may take various forms; such as a bayonet type arrangement, a threadedly engaged type arrangement or even a friction fit type arrangement. What is required is that the arrangement provides sufficient engaging force to hold the cartridge 12 and chamber 14 together, and overcome the biasing force applied to the stoppers 60 of the valve arrangement 56, to open the flow path.

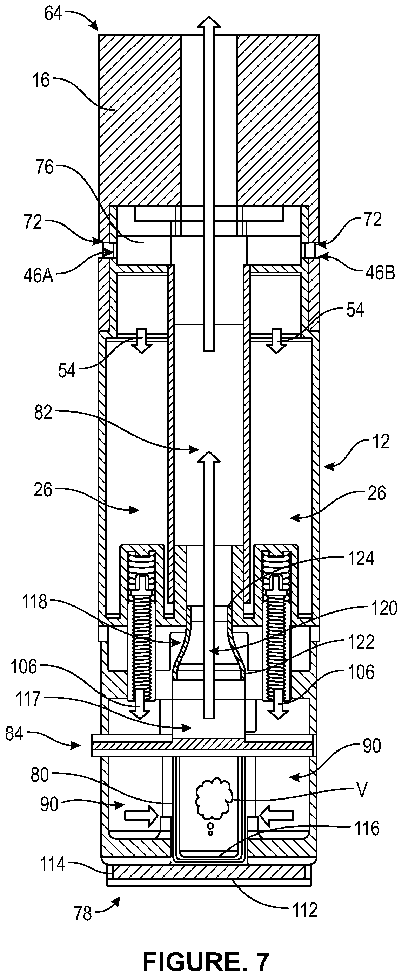

Turning now to Figure 7. The ultrasonic oscillation arrangement 78 includes a piezoelectric disc 112 seated in a flexible sleeve 114 which is in proximity to the wick 80.

The wick 80 is placed in contact with an atomization surface 116 of the piezoelectric disc 112. The wick is formed to have a capillary action to draw liquid placed in contact with the wick, and/or held in the inner chamber 90, to the atomization surface.

Unlike the use of a normal absorbent material, such as cotton, the capillary wick 80 will draw the liquid to be atomized to the atomization surface 116, irrespective of the orientation of the device 10 itself.

Therefore, certain absorbent materials may allow liquid to drain away from the atomization surface, say for example where the device is completely inverted. The liquid will drain through the material under the influence of gravity and away from the surface 116. In this scenario the device, although powered and functioning, could not be used in the ordinary sense. Oscillation of the piezoelectric ceramic disc 112 would still take place, but there would be no liquid to atomize.

By using the capillary wick 80, liquid will always be drawn to the atomization surface 116, even if the device 10 is completely inverted. Particularly useful for times when a user is horizontal, or lying down. The wick's construction, and physical properties ensure that saturation of the wick, by the liquid to be atomized, is always evenly distributed throughout the wick. Therefore, liquid cannot drain away (or in any direction) based on the orientation of the device 10.

To feed an amount of liquid to the inner chamber 90 for atomization, requires that the stoppers 60 of the valve arrangements 56 be moved against the biasing force applied by the biasing means 58 to open the liquid path 106.

This action is made possible by engaging the cartridge12 with the chamber 14, while the member 16 is twisted to an open position, to release the vacuum and allow the liquid, held in the reservoir 26, to flow through the liquid path 106, over pistons 102 and through holes 104 to the inner chamber 90.

When a sufficient amount of liquid has entered the inner chamber 90, the member 16 may be twisted to the sealing position, to thus restore the vacuum and hold the liquid within reservoir 26.

The liquid now in the inner chamber 90 will then meet the wick 80 and be drawn to the atomization surface 116.

The ultrasonic oscillation arrangement 78 is interconnected to the energy storage arrangement 94 and computer 98. The computer controls and regulates the oscillation of the piezoelectric disc 112. Thus, when the device is activated the liquid brought into contact with the atomization surface 116 is atomized to form a vape (V).

The chamber 14 includes at least one air inlet port 84 for supplying air from the surroundings to the inner chamber 90. The air supplied from the port is directed away from the atomization surface 116, and toward the direction of the inhalation channel 82.

In the current embodiment the inlet port 84 is shown as a tubular body having a cut-out portion located over, and orientated away from, the atomization surface 116.

The inhalation channel 82 is positioned over the atomization surface 116, such that any vape generated by the oscillation arrangement 78 is fed to the channel.

Air introduced through the port 84 mixes with the vape being generated from the atomization surface 116. The vape/air fluid mixture then passes into the inhalation channel 82, when in use.

The inhalation channel 82 extends from the chamber 14, through the internal bore 30 of the cartridge 12, and through the internal orifice 66, terminating at the trailing end 64 of the member 16. The channel additionally includes a mouth piece (not shown) at the trailing end.

The inhalation channel 82 includes a frustoconical body 118. The body includes an internal passage 120, aligned in the similar direction as the inhalation channel 82, having a proximal end 122 and a distal end 124. The distal end having a diameter less than that of the proximal end, such that the internal passage reduces in diameter over the bodies length.

The frustoconical body 118 is positioned in alignment with the atomization surface 116, wherein the proximal end 122 is nearer the surface. As shown in Figure 7 the air inlet port 84 is positioned between the atomization surface and the frustoconical body.

The vape/air fluid mixture which is passes into the inhalation channel 82 is met by the frustoconical body 118. The reducing internal passage 120 increases the pressure of the fluid mixture which, in turn accelerates movement of the fluid mixture through the channel and toward the mouth piece.

Figure 7 shows slots 72 of the member 16 in alignment with the ducts 46 (A and B, respectively) of the cartridge 12. When in alignment, air from the surroundings may flow to the reservoir 26.

The cartridge 12 and chamber 14 may be disengaged. When disengagement occurs, the pistons 102 are withdrawn from abutment with the stoppers 60 of the valve arrangement 56. The stoppers move to the sealing position under the force of the biasing means 58 and the reservoir 26 is sealed, thus preventing leakage of liquid from the reservoir.

Different cartridges 12 may be used interchangeably with the chamber 14, without having to completely or partially discharge the reservoir 26 from liquid. Each cartridge used and/or interchanged with the chamber is specifically coded and identifiable to the chamber through the cartridge's microchip 126.

The microchip 126 is brought into contact with the contact panel 86 when the cartridge 12 and chamber 14 are engaged. The panel provides communication between the data stored on the microchip and the computer 98.

The computer 98 and the energy storage arrangement form part of an electronic system for operating the device 10. The system includes, in addition to the computer 98 and the energy storage arrangement 94, a means for communication with an electronic device 130 and a GPS module 132.

The computer 98 operates the device 10. Such operation may include permitting power from the energy storage arrangement 94 to power the device 10 or preventing power from the energy storage arrangement to render the device unusable.

Powering the device 10, includes providing a current to the electronic oscillation arrangement 78. The arrangement, which includes the piezoelectric disc 112, cannot oscillate the disc without a current, and without oscillation, there is no atomization of a liquid at the atomization surface 116.

The operation may be dependent on the nature of the data contained on a microchip 126 and/or the physical location of the device. In addition, the computer 98 may operate the device according to any pre-programmed guidelines held within the computer's firmware or software.

The computer 98 may connect and communicate with an electronic device, through the means for communication with an electronic device 130. Typically, such communication will take place by connecting the device 10 to a program hosted on the electronic device.

The program may be used to operate the device 10, by passing commands to the computer 98.

In a use case, the communication will relate to determining the authenticity of the cartridge 12 being used with the device 10. The computer 98 is programmed to detect the use of a fake, or non-original, cartridge. And if such a fake cartridge was detected, the computer would prevent power to the device, rendering it unusable.

The computer 98 may also record data of the cartridge 12, such as make, model, number and even flavour, and volume of the cartridge. By recording and storing this data, the computer may operate the device 10.

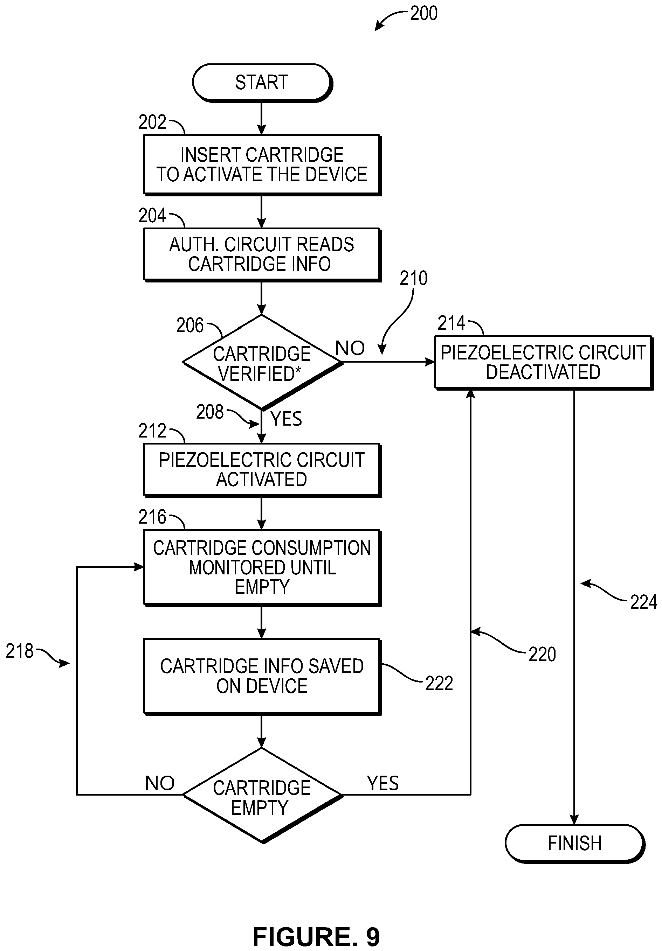

Figure 9 shows a flow diagram 200, which depicts the steps in which the computer 98 will operate the device 10, based on data stored on the microchip 126.

Step one 202 involves the cartridge 12 being engaged with the chamber 14. The engagement is such that the microchip 126 is brought into contact with the contact panel 86. Such contact provides a communication link between the microchip and the computer 98. Information relating to the cartridge's make, model, number, flavour and volume is read by the computer.

Step two 204 requires that the authenticity of the cartridge 12 is verified. Data specific to the cartridge in a coded form is communicated to the computer 98. The computer will run an algorithm designed specifically to identify cartridges with corresponding codes matching the algorithm to ensure authenticity of the cartridge.

Step three 206, involves authenticating the cartridge 12. If the code on the cartridge is compatible with the algorithms of the computer 98, a signal will be sent to activate the device 10. Such activation may involve permitting power from the energy storage arrangement 94 to the device, this is shown by arrow 208 (activate).

However, if the code is not compatible with the computer's 98 algorithm a signal will be sent to deactivate the device 10. Such deactivation may involve preventing power from the energy storage arrangement 94 to the device, this is shown by arrow 210 (deactivate). As referred to above, deactivation amounts to preventing power to the electronic oscillation arrangement 78. This in turn prevents oscillation of the piezoelectric disc 112 and no atomization can take place.

Step four 212 (activate). The signal to activate the device 10 may include a signal to activate the ultrasonic oscillation arrangement 78.

*Alternatively,* step four 214 (deactivate). The signal to activate the device 10 is not sent, and the ultrasonic oscillation arrangement 78 is not activated. In this instance, the operation 200 is ended, shown by arrow 224.

Only by replacing the cartridge 12 with a different cartridge will the operation be restarted. This serves as an anti-counterfeiting deterrent, allowing only original cartridges to function with the device 10.

Step five 216, the device 10 is used in accordance with ordinary normal use of the device. The computer 98 continually monitors the cartridge 12. The monitoring may include measuring the volume of liquid within the cartridge during use, and measuring rate of consumption, to determine when the cartridge is likely to be discharged of fluid.

If the cartridge still contains fluid and is currently in use with the device, the device will continue to operate in the ordinary normal way, this is depicted by arrow 218 (normal use).

When the cartridge is discharged, a signal will be sent to the computer 98 to this effect. Such a signal will result in the deactivation of the ultrasonic oscillation arrangement 78 and the end of the device 10 operation. This is depicted by arrow 220 (discontinued use).

Step six 222, during normal ordinary use of device 10, information related to the cartridge 12 is saved and stored on the computer 98. The information may be used in operating this, or other, cartridges.

The information stored on the computer 98, may include the make, model and volume of the reservoir and the nature of the liquid as it relates to a cartridge 12. Additionally, the information stored may also include the amount of liquid which has been discharged from the reservoir 26 during the use of the device 10.

By measuring the amount of liquid discharged, the computer 98 may predict, based on the number of times the device 10 is activated, how much liquid is remaining in the reservoir 26. The amount of liquid remaining provides a guideline as to how much longer the device may be used with the cartridge, 12 before the reservoir is completely discharged and when operation must be prevented.

When a cartridge 12 is removed, prior to all the liquid being discharged in the reservoir 26, the computer 98 will create a memory data entry to record the amount of liquid which remained in the cartridge. Once the cartridge is re-inserted, and normal use continues, the computer will recall the memory data of the amount of liquid remaining in the reservoir.

Operation of the device 10 will then only be permitted for as long as the remaining liquid would take to be depleted. Once this remaining fluid is depleted, the operation will be prevented.

This memory data recall will act as a tamper deterrent, wherein any additional liquid inserted to the reservoir 26 will not be used. Examples may include where a user attempts to refill the reservoir using a syringe filled with liquid, to increase the use of a particular cartridge 12.

The computer 98 may also operate the device 10 by determining the physical location of the device, using either the GPS module 132, or the GPS module of the electronic device. Locating the device within certain predefined geo-locations results in the computer preventing power from the energy storage arrangement 94 to power the device. In such a scenario, the operation 200 of the device 10 will be similar to that depicted in Figure 8.

Step three 206 will be replaced with a location confirmation relative to certain rules surrounding a predefined geolocation. Depending on the location of the device within the geolocation, the computer may operate the device 10 according to step four 212 (activate) as depicted by arrow 208 (activate) or *alternatively* step four 214 (deactivate) as depicted by arrow 210 (deactivate).

Over time, the computer 98 may require software updates to its processing systems.

Software updates may be communicated to the computer 98 in different ways. One such way to provide for an update is when the means for communicating with an electronic device 130 connects to the program on said electronic device. The program may contain the update and communicate this to the computer.

A further way in which an update may take place would be when the update data/information is contained on the microchip 126 of a cartridge 12 inserted into the device. The information is then transferred through the direct communication which is created when the microchip contacts the contact panel 86. The flow of data may then take place.

An even further way in which the computer 98 may be updated would be to place the contact panel 86 in contact with a dedicated updating system.

The dedicated updating system may be in any form which creates a physical connection between the contact panel 86 and an electronic device which contains the update. Common examples of such devices include a docking station, or an electronic cable connected to an electronic device, such as a computer.

Once the physical connection is made, the data, or update, may be transferred to the computer 98, to update the computer.

While certain embodiments have been selected to illustrate the present invention, and specific examples have been described herein, it will be obvious to those skilled in the art that various changes and modifications may be aimed to in the specification. It will, therefore, be understood by those skilled in the art that the particular embodiments of the invention presented here are by way of illustration only and are not meant to be in any way restrictive.

## Claims

1. A personal ultrasonic atomizer device (10) comprising:
a cartridge (12) having a reservoir (26) for holding a liquid to be atomized;
a sonication chamber (14), placed in fluid communication with the reservoir (26), and
a member (16) for controlling the amount of liquid flow into the chamber, wherein:
the reservoir (26) includes a first opening (50) for providing air passage from the surroundings to the interior of the reservoir (26) and a first aperture (52) for providing a flow path from the interior of the reservoir (26) to the sonication chamber (14);
the member (16) is engaged with the cartridge (12) to be movable between a sealed position, at which the opening (50) is closed, and a flow position, at which the opening (50) is open;
liquid held in the reservoir (26) is discharged to the sonication chamber (14) once the member (16) is in the flow position, and liquid held in the reservoir (26) remains in the reservoir (26) when the member (16) is in the sealed position, and
at least a first slot (72) through the member (16) provides air passage from the surroundings, wherein:
the slot (72) is aligned with the opening (50) when the member (16) is in the flow position, and wherein the slot (72) is misaligned with the opening (50) when the member (16) is in the sealed position, and optionally wherein the sealed position is an air-tight seal.

2. The device (10) as claimed in claim 1, wherein the member (16) is movable relative to the cartridge (12), between the open and the sealed position, in a lateral and/or vertical and/or twist manner.

## Patentansprüche

1. Persönliche Ultraschallzerstäubervorrichtung (10), umfassend:
eine Kartusche (12), die einen Vorratsbehälter (26) zum Halten von einer zu zerstäubenden Flüssigkeit aufweist;
eine Beschallungskammer (14), die in Flüssigkeitsverbindung mit dem Vorratsbehälter (26) steht, und
ein Element (16) zum Steuern der Flüssigkeitsmenge, die in die Kammer fließt, wobei:
der Vorratsbehälter (26) eine erste Öffnung (50) zum Bereitstellen eines Luftdurchgangs von der Umgebung in das Innere des Vorratsbehälters (26) und eine erste Öffnung (52) zum Bereitstellen eines Durchflusswegs von dem Inneren des Vorratsbehälters (26) zu der Beschallungskammer (14) aufweist;
das Element (16) mit der Kartusche (12) verbunden ist, um zwischen einer abgedichteten Position, in der die Öffnung (50) geschlossen ist, und einer Durchflussposition, in der die Öffnung (50) offen ist, beweglich zu sein;
Flüssigkeit, die in dem Vorratsbehälter (26) gehalten wird, in die Beschallungskammer (14) abgelassen wird, sobald das Element (16) in der Durchflussposition ist, und Flüssigkeit, die in dem Reservoir (26) enthalten ist, verbleibt im Vorratsbehälter (26) verbleibt, wenn das Element (16) in der abgedichteten Position ist, und
mindestens ein erster Schlitz (72) durch das Element (16) den Luftdurchgang aus der Umgebung bereitstellt, wobei:
wobei der Schlitz (72) mit der Öffnung (50) ausgerichtet ist, wenn das Element (16) in der Durchflussposition ist, und wobei der Schlitz (72) nicht mit der Öffnung (50) ausgerichtet ist, wenn das Element (16) in der abgedichteten Position ist, und optional wobei die abgedichtete Position eine luftdichte Abdichtung ist.

2. Vorrichtung (10) nach Anspruch 1, wobei das Element (16) relativ zu der Kartusche (12) zwischen der offenen und der abgedichtete Position, in einer seitlichen und/oder vertikalen und/oder drehenden Weise beweglich ist.

## Revendications

1. Dispositif (10) personnel d'atomisation ultrasonique comprenant :
une cartouche (12) ayant un réservoir (26) destiné à contenir un liquide à pulvériser ;
une chambre de sonification (14), placée en communication fluide avec le réservoir (26), et
un élément (16) pour commander la quantité de liquide entrant dans la chambre, dans lequel :
le réservoir (26) comporte une première ouverture (50) pour assurer le passage de l'air de l'environnement vers l'intérieur du réservoir (26) et une première ouverture (52) pour assurer une voie d'écoulement de l'intérieur du réservoir (26) vers la chambre de sonification (14) ;
l'élément (16) est en prise avec la cartouche (12) pour être mobile entre une position scellée, dans laquelle l'ouverture (50) est fermée, et une position d'écoulement, dans laquelle l'ouverture (50) est ouverte ;
le liquide contenu dans le réservoir (26) est déchargé dans la chambre de sonication (14) une fois que l'élément (16) est en position d'écoulement, et le liquide contenu dans le réservoir (26) reste dans le réservoir (26) lorsque l'élément (16) est en position scellée, et
au moins une première fente (72) à travers l'élément (16) permet le passage de l'air provenant de l'environnement, dans lequel :
la fente (72) est alignée avec l'ouverture (50) lorsque l'élément (16) est en position d'écoulement, et dans laquelle la fente (72) est désalignée avec l'ouverture (50) lorsque l'élément (16) est en position scellée, et éventuellement dans laquelle la position scellée est un joint étanche à l'air.

2. Dispositif (10) selon la revendication 1, dans lequel l'élément (16) est mobile par rapport à la cartouche (12), entre la position ouverte et la position scellée, de manière latérale et/ou verticale et/ou en torsion.
